(19) Europäisches Patentamt
European Patent Office
Office européen des brevets

(11) Veroffentlichungsnummer **0 008 342**
**B1**

# (12) EUROPÄISCHE PATENTSCHRIFT

(45) Veroffentlichungstag der Patentschrift 04.11.81

(51) Int Cl³ C 12 Q 1/52

(21) Anmeldenummer 79102249.4

(22) Anmeldetag 03.07.79

## (54) Verfahren und Reagenz zur Bestimmung von Glutamat-oxalacetat-transaminase und Glutamat-pyruvat-transaminase.

(30) Prioritat 08.08.78 DE 2834706

(43) Veroffentlichungstag der Anmeldung 05.03.80 Patentblatt 80/5

(45) Bekanntmachung des Hinweises auf die Patenterteilung 04.11.81 Patentblatt 81/44

(84) Benannte Vertragsstaaten BE CH DE FR GB IT LU NL SE

(56) Entgegenhaltungen
DE-A-1 673 294
DE-A1-2 431 779
DE-A1-2 448 230
US-A-3 953 294
US-A-4 024 021

(73) Patentinhaber BOEHRINGER MANNHEIM GMBH, Sandhofer Strasse 112-132 Postfach 31 01 20, D-6800 Mannheim 31-Waldhof (DE)

(72) Erfinder Deneke, Ulfert, Dr., Ammerhöfe 80 1/2, D-8123 Peissenberg (DE)
Erfinder Stahl, Peter, Dr., Jägerstrasse 12, D-8131 Bernried (DE)
Erfinder Schneider, Walter, Geistbühelstrasse 27, D-8120 Weilheim (DE)

(74) Vertreter Weickmann, Heinrich, Dipl.-Ing Patentanwälte Dipl.Ing.H.Weickmann et al, Dipl.Phys.Dr.K.Fincke Dipl.Ing.F.A.Weickmann Dipl.Chem.B.Huber, Dr.-Ing.H.Liska Möhlstrasse 22, D-8000 München 86 (DE)

EP 0 008 342 B1

Anmerkung Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebuhr entrichtet worden ist (Art 99(1) Europäisches Patentübereinkommen).

# Verfahren und Reagenz zur Bestimmung von Glutamatoxalacetat-transaminase und Glutamat-pyruvat-transaminase

Die Erfindung betrifft ein Verfahren zur Aktivitätsbestimmung der Enzyme Glutamat-oxalacetat-transaminase (GOT) und Glutamat-pyruvat-transaminase (GPT) sowie ein zur Durchführung dieses Verfahrens geeignetes Reagens. Das Verfahren eignet sich zur Bestimmung der genannten Enzyme in biologischen Flüssigkeiten, wie Serum, Urin oder in anderen Materialien.

Die große diagnostische Bedeutung der Bestimmung der genannten Transaminasen in Plasma, Serum, Geweben und anderem biologischen Material ist schon lange bekannt. Bei Diagnose und Differentialdiagnose, z. B. von Leber-, Herz- und Muskelerkrankungen sind diese Bestimmungen seit 1955 eingeführt und bis heute unumstritten, wie man den entsprechenden Lehrbüchern entnehmen kann (z. B. H. U. Bergmeyer, Methoden der enzymatischen Analyse, 3. Auflage, 1974, Verlag Chemie Weinheim, Band I, Seiten 6 bis 74). Es sind daher verschiedene Verfahren zur Bestimmung von GOT und GPT beschrieben worden, die jedoch alle mit Nachteilen behaftet sind.

Die genannten Transaminasen katalysieren folgende Reaktionen:

a) GPT

$$1)\ \text{Alanin} + \alpha\text{-Ketoglutarat} \xrightleftharpoons{\text{GPT}} \text{Pyruvat} + \text{Glutamat}$$

b) GOT

$$2)\ \text{Aspartat} + \alpha\text{-Ketoglutarat} \xrightleftharpoons{\text{GOT}} \text{Oxalacetat (OAA)} + \text{Glutamat}$$

Zur Bestimmung dieser Enzyme sind schon mehrere Methoden bekannt. So beschreiben K. S. Henley und H. M. Pollard (J. Lab. Clin. Med., 46 [1955], 785 bis 789) folgende Bestimmung der GPT-Aktivität: Die Reaktion 1) wird mit Alanin und α-Ketoglutarat (α-KG) durchgeführt und die Bildung des Pyruvats wie folgt gemessen:

$$3)\ \text{Pyruvat} + \text{NADH} + \text{H} \xrightarrow{\text{LDH}} \text{NAD}^+ + \text{Lactat}$$

LDH = Lactatdehydrogenase

Die Abnahme der Absorption von NADH pro min, gemessen im UV-Licht bei 365 nm, ist Meßsignal. Einen analogen Test auf GOT beschreibt A. Karmen (J. Clin. Invest., 34 [1955], 131 bis 133). Hierbei wird das nach Gleichung 2) gebildete Oxalacetat (OAA) wie folgt gemessen:

$$4)\ \text{OAA} + \text{NADH} + \text{H}^+ \xrightarrow{\text{MDH}} \text{Malat} + \text{NAD}^+$$

MDH = Malatdehydrogenase

Die Abnahme der Absorption von NADH pro min, gemessen bei 365 nm im UV-Licht, ist Meßsignal. Eine abgewandelte Methode ist in DE-A-2 448 230 beschrieben.

Ein anderes Verfahren, das sowohl zum Nachweis von GPT als auch von GOT geeignet sein soll, beschreiben S. Reitmann und S. Fraenkel in Am. J. Clin. Path., 28 (1957), 56 bis 63. Hierbei werden das durch GPT nach Gleichung 1) gebildete Pyruvat bzw. das durch GOT nach Gleichung 2) gebildete OAA gleichzeitig mit dem im Rest vorhandenen α-Ketoglutarat mit Dinitrophenylhydrazin zu den entsprechenden farbigen Hydrazonen umgesetzt, welche nach Alkalisierung aufgrund ihrer unterschiedlichen Absorption zwischen 500 und 550 nm gemeinsam bestimmt werden können. Die Berechnung ist kompliziert, aber möglich, weil das Hydrazon von α-Ketoglutarat einerseits und die Hydrazone von Pyruvat bzw. OAA andererseits, im Meßbereich unterschiedliche Extinktionen aufweisen.

Man kann das nach Gleichung 2) gebildete Oxalacetat aber auch mit Diazoniumsalzen zu einem Farbstoff kuppeln, der dann photometrisch ausgewertet werden kann. Ein solches Verfahren beschreibt A. L. Babson (Clin. Chem., 6 [1960], 394). Hiermit läßt sich die GOT-Aktivität messen (vgl. auch DE-A-1 673 294). Zur Messung der GPT-Aktivität benutzen T. Matsuzawa und N. Katunuma (Anal. Biochem., 17 [1966], 143 bis 153) die Bildung von α-KG nach Gleichung 1). Dieses α-KG überführen sie durch Zugabe von Aspartat und kristalliner GOT nach Gleichung 2) in OAA und messen dann dessen Bildung mit der Diazoniumsalz-Kupplung nach A. C. Babson. Die Menge des gebildeten AZO-Farbstoffs kann im sichtbaren Licht gemessen werden und ist ein Maß für die GPT- bzw. GOT-Aktivität in der Probe.

U. Lippi und G. Guidi (Clin. Chim. Acta, 28 [1970], 431 bis 437) maßen die GPT und GOT mit Hilfe des in Gleichung 1) und 2) freigesetzten Glutamats, indem sie folgende zwei Indikatorreaktionen nachschalteten:

$$5)\ \text{Glutamat} + \text{NAD}^+ \xrightarrow{\text{GlDH}} \alpha\text{-KG} + \text{NH}_3 + \text{NADH} + \text{H}^+$$

$$6)\ NADH + INT^+ \xrightarrow{PMS} Formazan + NAD^+$$

GIDH = Glutamatdehydrogenase;
PMS = Phenazinmethosulfat;
INT = 2-(p-Jodophenyl)-3-(p-nitrophenyl)-5-phenyl-tetrazoliumchlorid.

Ähnlich wird gemäß DE-A-2 431 779 und US-A-4 024 021 verfahren.

R. B. Domecq, M. Carta und E. F. de Armony (Biochim. Clinica, 2 [1971], 25 bis 36) kombinierten zur Bestimmung von GOT die Reaktionsfolge von Gleichung 2) und 4) mit Gleichung 6). Dadurch wird die Abnahme der NADH-Menge, gemessen als Formazanfarbstoff, im jeweiligen Test zur Meßgröße. Sie kann im sichtbaren Licht verfolgt werden und ist ebenfalls ein Maß für die Aktivität an GOT in der Probe. Für GPT beschreiben die Autoren ein analoges Verfahren (Biochim. Clinica, 2 [1971], 102 bis 111).

Alle hier beschriebenen Testverfahren zur Bestimmung der Aktivitäten der GPT und GOT weisen verschiedene gravierende Mängel auf, die ihre Anwendung verteuern oder erschweren. So erlauben die Verfahren nach Henley und Pollard bzw. nach A. Karmen zwar die Messung der Extinktionsänderung pro min von NADH, was vorteilhaft ist, es muß aber ein UV-Test angewandt werden. Wie dem Fachmann bekannt ist, sind optische Meßeinrichtungen zur Messung im UV-Licht aber besonders aufwendig und daher teurer als Meßanordnungen, die Absorptionsänderungen im sichtbaren Licht messen können. Ein weiterer schwerwiegender Mangel ist die Tatsache, daß eine Abnahme der NADH-Extinktion gemessen werden muß. In einem solchen Test kann aus meßtechnischen Gründen nur eine begrenzte Anfangskonzentration an NADH vorhanden sein. Das hat zur Folge, daß die hier verwendeten Indikatorenzyme LDH bzw. MDH nicht mit ihrem Substrat NADH gesättigt sind und demnach nicht maximale Reaktionsgeschwindigkeit erreichen. Das muß durch erhöhten Enzymeinsatz ausgeglichen werden. Dies ist nicht nur unwirtschaftlich, sondern beinhaltet auch die Gefahr, daß andere, störende Enzymaktivitäten in größerem Maße in das Testsystem eingeschleppt werden. Ein weiterer Nachteil der niedrigen NADH-Konzentration ergibt sich daraus, daß sowohl an Analysenautomaten als auch beim manuellen Test zwischen Zugabe der Probe und Beginn der Messung eine gewisse Zeit vergeht. Ist nun eine große Aktivität der GPT oder GOT in der Probe vorhanden, so wird bereits ein großer Teil des NADH vor Beginn der Messung verbraucht. Deswegen mißt man gerade bei stark erhöhten GPT- oder GOT-Werten, die Hinweise auf ein pathologisches Geschehen im Körper liefern, zu niedrige oder keine Aktivität. Dies ist ein schwerer Mangel dieser Bestimmungen.

Bei der Methode nach Reitman und Fraenkel werden die gebildeten Hydrazone im sichtbaren Licht bestimmt. Das ermöglicht die Anwendung einfacher und damit preisgünstiger photometrischer Einrichtungen. Dafür haften ihr andere schwerwiegende Mängel an. Sie ist sehr unempfindlich und benötigt deswegen die sehr lange Inkubationszeit von einer Stunde. Erst dann hat sich genug Pyruvat bzw. Oxalacetat gebildet, so daß die Hydrazonbildung eingeleitet werden kann. Diese benötigt eine weitere Inkubationszeit. Außerdem wird das im Testansatz erforderliche α-KG ebenfalls zu einem gefärbten Hydrazon umgesetzt. Dadurch ergeben sich für die Auswertung dieses Tests äußerst komplizierte Verhältnisse, da gleichzeitig die Abnahme von α-KG-Hydrazon und die Zunahme von Pyruvat- bzw. OAA-Hydrazon berücksichtigt werden müssen. Dem Fachmann ist daher schon seit langem bekannt, daß bei dieser Methode Fehler von 20 bis 30% hingenommen werden müssen.

Grundsätzlich ähnliche Probleme ergeben sich bei den Methoden nach Babson bzw. nach Matsuzawa und Katunuma. Zwar kann auch hier im sichtbaren Licht gemessen werden und weil α-KG mit Diazoniumsalz nicht interferiert, ist die Auswertung wesentlich einfacher; aber auch hier muß zunächst OAA bzw. Pyruvat durch längere Inkubation erzeugt werden. Anschließend muß die Farbbildung in einem weiteren Inkubationsschritt durchgeführt werden. Nach Matsuzawa und Katunuma wird der entstandene Azofarbstoff noch durch die unangenehm zu handhabende Salzsäure stabilisiert. Weiter ist dem Fachmann bekannt, daß viele Ketoverbindungen ähnlicher Struktur mit dem Diazoniumsalz zu Farbstoffen kuppeln, die die Bestimmung stören, weil sie zu hohe Werte vortäuschen. Diese Fehler müssen erst durch einen Leerwert eliminiert werden. So enthält z. B. Serum immer wechselnde Mengen Acetessigsäure, die Oxalacetat, das sich erst bei der GPT- bzw. GOT-Reaktion bilden soll, vortäuscht.

Die Methode nach Lippi und Guidi mißt ebenfalls mit sichtbaren Bereich. Sie benötigt nur noch einen Inkubationsschritt, der allerdings mit einer Dauer von 45 min ebenfalls sehr lang ist. Auch muß die Reaktion dann mit der giftigen und gefährlichen Salzsäure gestoppt werden. Ein weiterer Nachteil ist, daß im Serum relativ große Mengen an Glutaminsäure enthalten sind. Sie täuscht gemäß Gleichung 5) in beiden Tests GOT bzw. GPT vor. Deshalb muß auch hier ein Leerwert mitgeführt werden.

Das Verfahren von Domecq et al. benötigt ebenfalls zwei Inkubationsschritte. Da es letztlich die Abnahme von NADH, entsprechend der Methode von Henley und Pollard bzw. von Karmen mißt, gelten hier die gleichen Nachteile, wie dort aufgezeigt. Sie treten noch stärker hervor, weil aus meßtechnischen Gründen noch weniger NADH eingesetzt werden muß. Vorteilhaft ist die Messung im

sichtbaren Licht. Auch kann hier der völlige Verbrauch von NADH durch zu hohe Aktivitäten in der Probelösung nicht übersehen werden. Insgesamt aber ist, wie dem Fachmann ohne weiteres ersichtlich, das Verfahren sehr aufwendig und störanfällig, da es höchste Ansprüche an die präzise Dosierung von NADH in jedem Meßwert stellt.

Der Erfindung liegt daher die Aufgabe zugrunde, ein Verfahren zur Bestimmung der Aktivität der beiden genannten Transaminasen zu schaffen, welches die Nachteile der bekannten Verfahren nicht aufweist. Insbesondere ist es ein Ziel der Erfindung, ein derartiges Verfahren zu schaffen, welches kurze Meßzeit und nur eine Inkubation erfordert, die Absorptionsänderung pro min mißt, zu einer Absorptionszunahme und nicht zu einer -abnahme führt und auch eine Messung im sichtbaren Licht gestattet, damit einfachere photometrische Einrichtungen benutzt werden können.

Gelöst wird diese Aufgabe erfindungsgemäß durch ein Verfahren zur Bestimmung von Glutamat-oxalacetat-transaminase oder Glutamat-pyruvat-transaminase durch Umsetzung von Oxalacetat bzw. Pyruvat mit Glutamat unter Bildung von α-Ketoglutarat in gepufferter Lösung, welches dadurch gekennzeichnet ist, daß man gebildetes α-Ketoglutarat mit γ-Aminobutyrat in Gegenwart von γ-Aminobutyrat-transaminase umsetzt unter Bildung von Succinat-semialdehyd, mit letzterem NADP in Gegenwart von Succinat-semialdehyd-dehydrogenase zu NADPH reduziert und dieses entweder direkt mißt oder mit einem Tetrazoliumsalz und einem Elektronenüberträger in einen Formazanfarbstoff überführt und diesen mißt.

Das Prinzip des erfindungsgemäßen Verfahrens besteht in der Kombination der Reaktionen von Gleichung 1) bzw. 2) mit nachstehenden Gleichungen 7) und 8):

$$\text{7)}\quad \alpha\text{-KG} + \gamma\text{-Aminobutyrat} \xrightleftharpoons{\text{GAB—GT}} \text{Succinat-semialdehyd} + \alpha\text{-Glutamat}$$

GAB—GT = γ-Aminobutyrat-transaminase

$$\text{8)}\quad \text{Succinat-semialdehyd} + NADP^+ \xrightleftharpoons{\text{SS—Al—DH}} \text{Succinat} + NADPH + H$$

SS—Al—DH = Succinat-semialdehyd-dehydrogenase

Diese Gleichungen sind bekannt aus J. Biol. Chem., 234 (1958), 932 bis 940. Überraschenderweise wurde nunmehr gefunden, und hierauf beruht die Erfindung, daß es möglich ist, diese Reaktionen 7) und 8) mit den Gleichungen 1) und 2) über die α-KG-Bildung zu koppeln. Hierdurch wird die Absorptionszunahme pro Zeiteinheit durch das gebildete NADPH zum Maß der Aktivität von GPT bzw. GOT. Eine derartige Koppelung wurde bisher nicht für möglich gehalten, wie schon allein daraus hervorgeht, daß seit mehr als 20 Jahren nach einem befriedigenden Verfahren zur Bestimmung von GOT und GPT gesucht wird und auch die Reaktionen schon so lange bekannt sind.

Gemäß einer besonders vorteilhaften Ausführungsform dieses Verfahrens wird noch eine Reaktion nachgeschaltet, welche mit einem Tetrazoliumsalz in Gegenwart eines Elektronenüberträgers zur Rückoxidation des NADPH unter gleichzeitiger Bildung von gefärbtem Formazan führt, welches leicht im sichtbaren Licht gemessen werden kann. In einer bevorzugten Ausführungsform entspricht dies der nachstehenden Gleichung 9).

$$\text{9)}\quad NADPH + MTT + H \xrightarrow{\text{Diaphorase}} \text{Formazan} + NADP$$

MTT = 3-(4,5-Dimethyl-thiazolyl-2)-2,5-diphenyl-tetrazoliumbromid.

Die in Gleichung 9) als Elektronenüberträger aufgeführte Diaphorase wird bevorzugt. Es können jedoch auch andere Elektronenüberträger, die dem Fachmann bekannt sind, verwendet werden. Als sehr gut geeignet haben sich dabei insbesondere Phenazinmethosulfat (PMS) und Phenantrolinmethosulfat erwiesen. Diese Ausführungsform eignet sich auch gut für die Anwendung auf Teststreifen.

Das erfindungsgemäße Verfahren wird im allgemeinen bei pH-Werten zwischen 7 und 9,5 durchgeführt. Bei höheren und niedrigeren pH-Werten ergibt sich eine erhebliche Verlangsamung der Reaktion mit einer entsprechenden Verlängerung des Zeitbedarfs pro Test. Die besten Ergebnisse werden bei pH-Werten zwischen etwa 8 und 8,6 erzielt.

Geeignete Pufferkonzentrationen liegen zwischen 5 mMol/l und 0,5 Mol/l, entsprechend etwa 0,05 bis 6% Puffersalz im Test. Bevorzugt wird Puffer in einer Konzentration zwischen etwa 10 und etwa 100 mMol/l eingesetzt.

Die Art des verwendeten Puffers ist unkritisch. Wichtig ist allein, daß er in dem obengenannten Bereich puffert. So wurden beispielsweise vergleichbar gute Resultate mit Phosphatpuffer, Triäthanolaminpuffer, Glycinpuffer, Pyrophosphatpuffer, Trispuffer oder Imidazolpuffer erzielt. Die beiden letztgenannten Puffer werden aus meßtechnischen und Handhabungsgründen bevorzugt. Die übrigen, im erfindungsgemäßen Verfahren eingesetzten Reagentien und Enzyme können in einem weiten Konzentrationsbereich eingesetzt werden, ohne das Ergebnis der Messungen zu verändern. In keinem Falle ist es erforderlich für eines der Reagentien kritische enge Grenzen einzuhalten.

Als Tetrazoliumsalz wird das bereits erwähnte MTT bevorzugt. Es können aber auch andere

Tetrazoliumsalze verwendet werden. Gute Ergebnisse wurden insbesondere auch mit Nitroblau-tetrazoliumchlorid (NBT) und 2-(p-Jodophenyl)-3-(p-nitrophenyl)-5-phenyl-tetrazoliumchlorid (INT) erzielt. Die Auswahl des Tetrazoliumsalzes erfolgt unter Berücksichtigung der Löslichkeit des gebildeten Formazanfarbstoffs. Weist letzterer nämlich eine geringe Löslichkeit auf, so können bei hohen GOT- oder GPT-Aktivitäten Ausfällungen des Farbstoffs erfolgen, welche zu Meßschwierigkeiten führen. Aus diesem Grunde wird zweckmäßig bei Durchführung des erfindungsgemäßen Verfahrens unter Messung eines Formazanfarbstoffs ein oberflächenaktives Mittel zugesetzt, welches das Löslichkeitsverhalten des Farbstoffs verbessert. Beispiele für geeignete oberflächenaktive Mittel sind Desoxycholsäure, Saponin, Alkylaryl-polyäthylenglykol-ester und -äther, Sorbitanester, wie Sorbimacrogololeat, Polyäthylenglykollauryläther u. dgl. Die Konzentration an oberflächenaktivem Mittel liegt im allgemeinen zwischen 0,3 und 3% im Test.

Ein weiterer Gegenstand der Erfindung ist ein Reagens zur Bestimmung von GOT und GPT, welches dadurch gekennzeichnet ist, daß es $\gamma$-Aminobutyrat-transaminase (GAB-GT), Succinat-semialdehyd-dehydrogenase (SS-Al-DH), $\gamma$-Aminobutyrat, Glutamat, NADP, Puffer sowie entweder Oxalacetat oder Pyruvat und gegebenenfalls Tetrazoliumsalz, Elektronenüberträger und oberflächenaktives Mittel enthält.

Bei $\gamma$-Aminobutyrat, Glutamat, Oxalacetat und Pyruvat handelt es sich um die Salze der zugrunde liegenden Säuren mit Kationen, welche die enzymatische Reaktion nicht beeinträchtigen. Im allgemeinen werden Alkali-, Ammonium- oder Aminosalze bevorzugt, es können jedoch auch Magnesiumsalze und dergleichen verwendet werden.

Ein bevorzugtes Reagens dieser Art enthält

0,5 bis 20 U/ml SS-Al-DH,
0,5 bis 20 U/ml GAB-GT,
0,1 bis 50 mMol/l Pyruvat oder Oxalacetat,
0,02 bis 0,5 Mol/l Glutamat,
10 bis 300 mMol/l $\gamma$-Aminobutyrat,
0,2 bis 10 mMol NADP und
10 bis 100 mMol/l Puffer,

jeweils bezogen auf Konzentration im Test. Das Reagens kann in trockener Form oder in Form einer Lösung vorliegen. Es können sämtliche Bestandteile gemischt oder getrennt vorliegen.

Vorzugsweise enthält ein derartiges Reagens zusätzlich

0,02 bis 1 U/ml Diaphorase,
0,02 bis 0,5 mMol/l MTT, INT oder NBT und
0,3 bis 3% oberflächenaktives Mittel.

Die Diaphorase kann dabei auch durch nichtenzymatisch wirkende Elektronenüberträger ersetzt werden. Dieses Reagens eignet sich auch besonders zur Imrägnierung bzw. Inkorporierung in Trägermaterialien für die Herstellung von Teststreifen.

Die erfindungsgemäße Bestimmung verläuft rasch. Eine Inkubationsdauer zwischen 1 und 5, vorzugsweise etwa 2 min, reicht vollständig aus. Nach dem Start der Reaktion durch Zugabe der zu untersuchenden Probelösung ist die Messung nach wenigen min beendet. In der Regel wird die Extinktiondifferenz pro min gemessen. Hierzu genügen einige Meßwerte, die innerhalb 1 oder 2 min aufgenommen werden. Der Einfachheit halber werden zwei bis vier Meßwerte in Minutenabständen genommen.

Das erfindungsgemäße Verfahren zeichnet sich durch hohe Genauigkeit, geringen Zeitbedarf, geringe Störungsanfälligkeit und Durchführbarkeit mit einfachen photometrischen Einrichtungen aus. Da die Messung der Absorptionsänderung pro min erfolgt, bleibt die Meßzeit kurz. Es ist auch nur eine einzige Inkubation erforderlich. Ein wesentlicher Vorteil liegt darin, daß die Absorptionsänderung eine Absorptionszunahme darstellt, so daß der Meßparameter nicht in begrenzten Mengen eingesetzt werden muß und keine falschen Werte von zu hohen Aktivitäten an GPT oder GOT vorgetäuscht werden können, wie dies bei Verfahren der Fall ist, bei denen eine Absorptionszunahme erfolgt.

In den nachfolgenden Beispielen werden folgende Abkürzungen verwendet:

| | |
|---|---|
| GPT | Glutamat-pyruvat-transaminase |
| GAB-GT | $\gamma$-Aminobutyrat-$\alpha$-ketoglutarat-transaminase |
| SS-Al-DH | Succinatsemialdehyd-dehydrogenase |
| GOT | Glutamat-oxalacetat-transaminase |
| NADP | Nicotinamid-adenin-dinucleotidphosphat |
| NADPH | Nicotinamid-adenin-dinucleotid-phosphat, reduziert |
| MTT | 3-(4,5-Dimethylthiazolyl-2)-2,5-diphenyl-tetrazoliumbromid |
| Tris | Tris(hydroxymethyl)-aminomethan |
| Triton® X100 | Alkylaryl-polyäthylenglycol-äther |

Die folgenden Beispiele erläutern die Erfindung weiter.

## Beispiel 1

### Nachweis von GPT; NADPH-Bildung als Meßsignal

Temperatur 25°C, Wellenlänge 365 nm, Testvolumen 3 ml, 1 cm Küvetten.

| Ausgangslösung | Menge | Konzentration im Test |
|---|---|---|
| Tris HCl, pH 8,3, 50 mMol/l (=06055%) | 1,5 ml | 25 mMol/l (=0,30275 %) |
| NADP, 27 mMol/l (= 2 %) γ-Aminobutyrat, 0,9 mMol/l | 0,1 ml | 0,9 mMol/l (= 0,067 %) |
| pH 8,3 (= 9 %) | 0,2 ml | 60 mMol/l (= 0,6 %) |
| Glutamat, 0,6 Mol/l, pH 8,3 (= 8,8%) | 0,5 ml | 0,1 Mol/l ( 1,47 %) |
| Natriumpyruvat, 0,55 Mol/l (= 6 %) | 0,03 ml | 5,4 mMol/l (= 0,06 %) |
| GAB-GT, 86 U/ml ) SS-Al-DH 133 U/ml) = 11 % Protein | 0,5 ml | 1,43 U/ml (= 0,183 %) 2,2 U/ml (= 0,183 %) |
| $H_2O$ | 0,02 ml | |
| mischen, starten mit Serum (GPT-haltig) | 0,5 ml | |

mischen, 2 min inkubieren, $E_1$ ablesen, nach genau 1, 2, 3 und 4 min $E_2$, $E_3$, $E_4$ und $E_5$ ablesen. Daraus das $\Delta$ E/min berechnen. Die GPT-Aktivität in der Probe berechnet sich nach

$$U/ml = \frac{E \cdot V}{\varepsilon \cdot v} = \Delta E \cdot 1{,}714$$

Beispiel 2

GPT-Bestimmung; Formazan-Bildung als Meßsignal.

Temperatur 25°C, Wellenlänge 578 nm, Testvolumen 3 ml, 1 cm Küvette.

In Küvette pipettieren:

| Ausgangslösung | Menge | Konzentration im Test |
|---|---|---|
| Tris HCl, pH 8,3, 50 mMol/l (= 0,6055 %), enthaltend 2 % Triton X 100 | 1,5 ml | Triton X100 (= 1 %)<br>Tris 25 mMol/l (= 0,30275 %) |
| NADP, 27 mMol/l (= 2 %) | 0,1 ml | 0,9 mMol/l (= 0,067 %) |
| γ-Aminobutyrat, 0,9 Mol/l (= 9 %) | 0,2 ml | 60 mMol/l (= 0,6 %) |
| Glutamat, 0,6 Mol/l (= 8,8 %) | 0,5 ml | 0,1 Mol/l (= 1,47 %) |
| Natriumpyruvat, 0,55 Mol/l (= 6 %) | 0,03 ml | 5,4 mMol/l (= 0,06 %) |
| MTT, 1,5 mMol/l (= 0,0621 %) | 0,2 ml | 0,1 mMol/l (= 0,00414 %) |
| GAB-GT, 86 U/ml (= 11 % Protein) | 0,05 ml | 1,43 U/ml (= 0,183 %) |
| SS-Al-DH, 133 U/ml (= 11 % Protein) | 0,05 ml | 2,2 U/ml (=0,183 %) |
| Diaphorase, 23 U/ml (= 0,5 % Protein) | 0,01 ml | 0,077 U/ml (= 0,0017 %) |
| $H_2O$ | 0,26 ml | |
| mischen, starten mit Serum (GPT-haltig) | 0,1 ml | |

mischen, 2 min inkubieren, $E_1$ ablesen, nach genau 1, 2, 3 und 4 min $E_2$, $E_3$, $E_4$ und $E_5$ ablesen, daraus das $\Delta E$/min berechnen. Die GPT-Aktivität in der Probe berechnet sich wie folgt:

$$U/ml \cdot \frac{E \cdot V}{\varepsilon \cdot v} = \Delta E \cdot 1{,}796$$

Beispiel 3

GOT-Bestimmung; NADPH-Bildung als Meßsignal

Meßtemperatur 25°C, Meßwellenlänge 365 nm, Testvolumen 3 ml, 1 cm Küvette.

In Küvette pipettieren:

| Ausgangslösung | Menge | Konzentration im Test |
|---|---|---|
| Imidazol · HCl, pH 7,6, 100 mMol/l (= 6,808 %) | 1,5 ml | 50 mMol/l (= 3,404 %) |
| NADP, 27 mMol/l (= 2 %) | 0,1 ml | 0,9 mMol/l (= 0,067 %) |
| γ-Aminobutyrat, 0,9 Mol/l, pH 8,3 (= 9 %) | 0,3 ml | 90 mMol/l (= 0,9 %) |
| Glutamat, 0,6 mMol/l, pH 8,3 (= 8,8 %) | 0,3 ml | 60 mMol/l (= 0,88 %) |
| Oxalacetat, 20,8 mMol/l (= 0,275 %) | 0,1 ml | 0,65 mMol/l (= 0,00917 %) |
| GAB-GT, 86 U/ml (= 11 % Protein) | 0,05 ml | 1,43 U/ml (= 0,183 %) |
| SS-Al-DH, 133 U/ml (= 11 % Protein) | 0,05 ml | 2,22 U/ml (= 0,183 %) |
| $H_2O$ | 0,1 ml | |
| mischen, starten mit Serum (GOT-haltig) | 0,5 ml | |

mischen, 2 min inkubieren, $E_1$ ablesen, nach genau 1, 2, 3 und 4 min $E_2$, $E_3$, $E_4$ und $E_5$ ablesen, daraus das $\Delta$ E/min berechnen. Die GOT-Aktivität in der Probe berechnet sich nach

$$U/ml = \frac{\Delta E \cdot V}{\varepsilon \cdot v} = \Delta E \cdot 1{,}717$$

Beispiel 4

Nachweis von GOT; Formazanbildung als Meßsignal

Temperatur 25°C, Meßwellenlänge 578 nm, Testvolumen 3 ml, 1 cm Küvetten.

| Ausgangslösung | Menge | Konzentration im Test |
|---|---|---|
| Imidazol · HCl, pH 7,6, 100 mMol/l (= 6,808 %) + 3 % Triton X 100 | 1,5 ml | Imidazol 50 mMol/l (= 0,30275 %) Triton X 100 (= 1,5 %) |
| NADP, 27 mMol/l (= 2 %) | 0,1 ml | 0,9 mMol/l (= 0,067 %) |
| γ-Aminobutyrat, 0,9 Mol/l, pH 8,3 (= 9 %) | 0,3 ml | 90 mMol/l (= 0,9 %) |
| Glutamat, 0,5 Mol/l, pH 8,3 (= 8,8 %) | 0,3 ml | 60 mMol/l (= 0,88 %) |
| Oxalacetat, 20,8 mMol/l (= 0,275 %) | 0,1 ml | 0,65 mMol/l (= 0,00917 %) |
| MTT, 1,5 mMol/l (= 0,0621 %) | 0,2 ml | 0,1 mMol/l (= 0,00414 %) |
| GAB-GT, 86 U/ml (= 11 % Protein) | 0,05 ml | 1,43 U/ml (= 0,183 %) |
| SS-Al-DH 133 U/ml (= 11 % Protein) | 0,05 ml | 2,22 U/ml (= 0,183 %) |
| Diaphorase, 23 U/ml (= 0,5 % Protein) | 0,01 ml | 0,077 U/ml (= 0,0017 %) |
| $H_2O$ | 0,24 ml | |
| mischen, starten mit Serum (GOT-haltig) | 0,05 ml | |

mischen, 2 min inkubieren, $E_1$ ablesen, nach genau 1, 2, 3 und 4 min $E_2$, $E_3$, $E_4$ und $E_5$ ablesen. Die GOT-Aktivität der Probe wie folgt berechnen:

$$U/ml = \frac{\Delta E \cdot V}{\varepsilon \cdot v} = \Delta E \cdot 3{,}593$$

Beispiel 5

Nachweis von GPT auf Teststreifen

Ein geeignetes Papier wird mit einer Lösung getränkt, die sämtliche Reagentien des Beispiels 2 enthält, vorsichtig getrocknet, auf ein geeignetes Trägermaterial fixiert, eingesiegelt und in Streifen geschnitten. Nach Eintauchen in Serum entsteht eine rotblaue Färbung, deren Intensität proportional zur GPT-Aktivität in der Probe ist. Die Auswertung kann durch Vergleich mit einer geeigneten Standardfarbskala erfolgen. Ebenso ist die Auswertung mit dem Reflexionsphotometer möglich.

Beispiel 6

Nachweis von GOT auf Teststreifen

Ein geeignetes Papier wird mit einer nach Beispiel 4 bereiteten Reagenslösung getränkt, vorsichtig getrocknet, auf ein geeignetes Trägermaterial fixiert, eventuell eingesiegelt und in Streifen geschnitten. Nach Eintauchen in Serum entsteht eine rotblaue Färbung, deren Intensität proportional zur GOT-Aktivität in der Probe ist. Die Auswertung kann durch Vergleich mit einer geeigneten Standardfarbskala erfolgen. Ebenso ist die Auswertung mit dem Reflexionsphotometer möglich.

**Patentansprüche**

1. Verfahren zur Bestimmung von Glutamat-oxalacetat-transaminase oder Glutamat-pyruvat-transaminase durch Umsetzung von Oxalacetat bzw. Pyruvat mit Glutamat unter Bildung von α-Ketoglutarat

in gepufferter Lösung, dadurch gekennzeichnet, daß man gebildetes α-Ketoglutarat mit γ-Aminobutyrat in Gegenwart von γ-Aminobutyrat-transaminase umsetzt unter Bildung von Succinat-semialdehyd, mit letzterem NADP in Gegenwart von Succinat-semialdehyd-dehydrogenase zu NADPH reduziert und dieses entweder direkt mißt oder mit einem Tetrazoliumsalz und einem Elektronenüberträger in einen Formazanfarbstoff überführt und diesen mißt.

2. Verfahren nach Anspruch 1, dadurch gekennzeichnet, daß als Elektronenüberträger Diaphorase, Phenantrolinmethosulfat oder Phenazinmethosulfat verwendet wird.

3. Verfahren nach Anspruch 1 oder 2, dadurch gekennzeichnet, daß als Tetrazoliumsalz 3-(4,5-Dimethylthiazolyl-2)-2,5-diphenyl-tetrazoliumbromid, Nitroblautetrazoliumchlorid oder 2-(p-Jodophenyl)-3-(p-nitrophenyl)-5-phenyl-tetrazoliumchlorid verwendet wird.

4. Verfahren nach einem der vorhergehenden Ansprüche, dadurch gekennzeichnet, daß ein pH-Wert zwischen 7 und 9,5 eingestellt wird.

5. Verfahren nach einem der vorhergehenden Ansprüche, dadurch gekennzeichnet, daß eine Pufferkonzentration zwischen 0,05 und 6% angewendet wird.

6. Reagens zur Bestimmung von Glutamat-oxalacetattransaminase oder Glutamat-pyruvat-transaminase, dadurch gekennzeichnet, daß es γ-Aminobutyrat-transaminase, Succinat-semialdehyd-dehydrogenase, γ-Aminobutyrat, Glutamat, NADP, Puffer, entweder Oxalacetat oder Pyruvat und gegebenenfalls Tetrazoliumsalz, Elektronenüberträger und oberflächenaktives Mittel enthält.

7. Reagens nach Anspruch 6, dadurch gekennzeichnet, daß es als Elektronenüberträger Diaphorase, Phenantrolinmethosulfat oder Phenazinmethosulfat enthält.

8. Reagens nach Anspruch 6, dadurch gekennzeichnet, daß es als Tetrazoliumsalz 3-(4,4-Dimethylthiazolyl-2)-2,5-diphenyl-tetrazoliumbromid, Nitroblau-tetrazoliumchlorid oder 2-(p-Jodophenyl)-3-(p-nitrophenyl)-5-phenyl-tetrazoliumchlorid enthält.

9. Reagens nach Anspruch 6, dadurch gekennzeichnet, daß es

0,5 bis 20 U/ml Succinat-semialdehyd-dehydrogenase,
0,5 bis 20 U/ml γ-Aminobutyrat-transaminase,
0,1 bis 50 mMol/l Pyruvat oder Oxalacetat,
0,02 bis 0,5 Mol/l Glutamat,
10 bis 300 mMol/l γ-Aminobutyrat,
0,2 bis 10 mMol/l NADP und
10 bis 100 mMol/l Puffer,

bezogen auf die Konzentration im Test, enthält.

10. Reagens nach Anspruch 9, dadurch gekennzeichnet, daß es zusätzlich

0,02 bis 1 U/ml Diaphorase,
0,02 bis 0,5 mMol/l 3-(4,5-Dimethyl-thiazolyl-2)-2,5-diphenyltetrazoliumbromid,
0,5 bis 5% oberflächenaktives Mittel,

bezogen auf Konzentration im Test, enthält.

11. Reagens nach einem der Ansprüche 6 bis 10, dadurch gekennzeichnet, daß es Trispuffer oder Imidazolpuffer enthält.

**Claims**

1. Process for the determination of glutamateoxalacetate transaminase or glutamate-pyruvate transaminase by the reaction of oxalacetate or pyruvate with glutamate with the formation of α-ketoglutarate in buffered solution, characterised in that one reacts α-ketoglutarate formed with γ-aminobutyrate in the presence of γ-aminobutyrate transaminase with the formation of succinate semialdehyde, reduces NADP with the latter in the presence of succinate-semialdehyde dehydrogenase to NADPH and either measures this directly or converts with a tetrazolium salt and an electron carrier into a formazane coloured material and measures this.

2. Process according to claim 1, characterised in that as electron carrier there is used diaphorase, phenanthroline methosulphate or phenazine methosulphate.

3. Process according to claim 1 or 2, characterised in that as tetrazolium salt there is used 3-(4,5-dimethylthiazolyl-2)-2,5-diphenyltetrazolium bromide, nitro blue tetrazolium chloride or 2-(p-iodophenyl)-3-(p-nitrophenyl)-5-phenyltetrazolium chloride.

4. Process according to one of the preceding claims, characterised in that a pH value is adjusted of between 7 and 9.5.

5. Process according to one of the preceding claims, characterised in that a buffer concentration is used of between 0.05 and 6%.

6. Reagent for the determination of glutamateoxalacetate transaminase or glutamate-pyruvate transaminase, characterised in that it contains γ-aminobutyrate transaminase, succinate-semialde-

hyde dehydrogenase, $\gamma$-aminobutyrate, glutamate, NADP, buffer, either oxalacetate or pyruvate and possibly tetrazolium salt, electron carrier and surface-active agent.

7. Reagent according to claim 6, characterised in that as electron carrier it contains diaphorase, phenanthroline methosulphate or phenazine methosulphate.

8. Reagent according to claim 6, characterised in that as tetrazolium salt is contains 3-(4,5-dimethylthiazolyl-2)-2,5-diphenyltetrazolium bromide, nitro blue tetrazolium chloride or 2-(p-iodophenyl)-3-(p-nitrophenyl)-5-phenyltetrazolium chloride.

9. Reagent according to claim 6, characterised in that it contains

0,5 to 20 U/ml succinate-semialdehyde dehydrogenase,
0,5 to 20 U/ml $\gamma$-aminobutyrate transaminase,
0,1 to 50 mMol/l pyruvate or oxalacetate,
0,02 to 0,5 mol/l glutamate,
10 to 300 mMol/l $\gamma$-aminobutyrate,
0,2 to 10 mMol/l NADP and
10 to 100 mMol/l buffer,

referred to the concentration in the test.

10. Reagent according to claim 9, characterised in that it additionally contains

0,02 to 1 U/ml diaphorase,
0,02 to 0,5 mMol/l 3-(4,5-dimethylthiazolyl-2)-2,5-diphenyltetrazolium bromide,
0,5 to 5% surface-active agent,

referred to the concentration in the test.

11. Reagent according to one of claims 6 to 10, characterised in that it contains tris buffer or imidazole buffer.

**Revendications**

1. Procédé pour le dosage de la glutamate-oxalacétate-transaminase ou de la glutamate-pyruvate-transaminase par réaction de l'oxalacétate ou respectivement du pyruvate avec le glutamate avec formation de $\alpha$-cétoglutarate en solution tamponnée, caractérisé en ce qu'on fait réagir l'$\alpha$-cétoglutarate formé avec le $\gamma$-aminobutyrate en présence de $\gamma$-aminobutyrate-transaminase avec formation de succinate-semialdéhyde, on réduit avec ce dernier le NADP, en présence de succinate-semialdéhyde-deshydrogénase, en NADPH et soit on mesure celui-ci directement soit on le transforme, au moyen d'un sel de tétrazolium et d'un agent de transfert d'électrons, en un colorant formazan et on mesure celui-ci.

2. Procédé selon la revendication 1, caractérisé en ce qu'on utilise, en tant qu'agent de transfert d'électrons, de la diaphorase, du méthosulfate de phénanthroline ou du méthosulfate de phénazine.

3. Procédé selon la revendication 1 ou 2, caractérisé en ce qu'on utilise, en tant que sel de tétrazolium, du bromure de 3-(4,5-diméthylthiazolyl-2)-2,5-diphényl-tétrazolium, du chlorure de nitrobleutétrazolium ou du chlorure de 2-(p-iodophényl)-3-(p-nitrophényl)-5-phényl-tétrazolium.

4. Procédé selon l'une des revendications précédentes, caractérisé en ce qu'on établit une valeur de pH comprise entre 7 et 9,5.

5. Procédé selon l'une des revendications précédentes, caractérisé en ce qu'on utilise une concentration en tampon comprise entre 0,05 et 6%.

6. Réactif pour le dosage de la glutamate-oxalacétate-transaminase ou de la glutamate-pyruvate-transaminase, caractérisé en ce qu'il contient de la $\gamma$-aminobutyrate-transaminase, de la succinate-semialdéhyde-deshydrogénase, du glutamate, du NADP, du tampon, soit de l'oxalacétate soit du pyruvate et éventuellement du sel de tétrazolium, de l'agent de transfert d'électrons et de l'agent tensio-actif.

7. Réactif selon la revendication 6 caractérisé en ce qu'il contient, en tant qu'agent de transfert d'électrons de la diaphorase, du méthosulfate de phénanthroline ou du méthosulfate de phénazine.

8. Réactif selon la revendication 6, caractérisé en ce qu'il contient, en tant que sel de tétrazolium, du bromure de 3-(4,5-diméthylthiazolyl-2)-2,5-diphényl-tétrazolium, du chlorure de nitrobleutétrazolium ou du chlorure de 2-(p-iodophényl)-3-(p-nitrophényl)-5-phényl-tétrazolium.

9. Réactif selon la revendication 6, caractérisé en ce qu'il contient

0,5 à 20 U/ml de succinate-semialdéhyde-deshydrogénase,
0,5 à 20 U/ml de $\gamma$-aminobutyrate-transaminase,
0,1 à 50 mmoles/l de pyruvate ou oxalacétate,
0,02 à 0,5 mole/l de glutamate,
10 à 300 mmoles/l de $\gamma$-aminobutyrate,

0,2 à 10 mmoles/l de NADP et
10 à 100 mmoles/l de tampon,

en rapport avec la concentration dans l'essai.

10. Réactif selon la revendication 9, caractérisé en ce qu'il contient en outre

0,02 à 1 U/ml de diaphorase,
0,02 à 0,5 mmole/l de bromure de 3-(4,5-diméthyl-thiazolyl-2)-2,5-diphényltétrazolium,
0,5 à 5% d'agent tensio-actif,

en rapport avec la concentration dans l'essai.

11. Réactif selon l'une des revendications 6 à 10, caractérisé en ce qu'il contient du tampon tris ou du tampon à l'imidazole.